# EUROPEAN PATENT SPECIFICATION

(11) **EP 2 687 215 B1**
(45) Date of publication and mention of the grant of the patent: **28.11.2018**
(21) Application number: 12176851.9
(22) Date of filing: 18.07.2012
(51) Int. Cl.: A61K 31/565, A61K 31/568, A61K 31/5685, A61K 31/57, A61K 45/06, A61P 5/24, A61K 31/19, A61K 31/375

(54) **Mesterolone pharmaceutical composition for dihydrotestosterone deficiencies in woman**
Pharmazeutischer Mesterolone für Dihydrotestosteronmängel bei der Frau
Mesterolone composition pharmaceutique pour les déficiences en dihydrotestostérone chez la femme

(43) Date of publication of application: 22.01.2014
(73) Proprietor: Debled, Georges, 11540 Sanlúcar de Barrameda, Cadiz (ES)
(72) Inventor: Debled, Georges, 11540 Sanlúcar de Barrameda, Cadiz (ES)
(74) Representative: Debled, Thierry

(56) References cited:
- WO-A1-03/039553
- CAVADAS L F ET AL: "Management of menopause in primary health care", ACTA MEDICA PORTUGUESA 2010 CENTRO EDITOR LIVREIRO DA ORDEM DOS MEDICOS PRT, vol. 23, no. 2, March 2010 (2010-03), pages 227-236, XP009165886, ISSN: 0870-399X
- Anonymous: "Proviron", , 30 December 2010 (2010-12-30), XP055282046, Retrieved from the Internet: URL:http://www.drugwiki.net/drugs/Proviron [retrieved on 2016-06-20]
- TRAISH A M ET AL: "Testosterone therapy in women with gynecological and sexual disorders: A triumph of clinical endocrinology from 1938 to 2008", JOURNAL OF SEXUAL MEDICINE, BLACKWELL PUBLISHING LTD., OXFORD, GB, vol. 6, no. 2, 1 January 2009 (2009-01-01) , pages 334-351, XP002607089, ISSN: 1743-6095, DOI: 10.1111/J.1743-6109.2008.01121.X

## Description

The invention covers certain indications for mesterolone in woman, in particular, for the treatment and prevention treatment of disorders associated with the deficiency in dihydrotestosterone in woman. More particularly, the present invention concerns the use of mesterolone in the treatment and prevention treatment of bladder neck fibrosis or sclerosis, urinary problems such as chronic cystitis, incontinence and urgencies in woman. In another of its aspects, the present invention concerns the use of mesterolone for use in the treatment and prevention treatment of dyspareunia in woman. According to the present invention, the mesterolone is administered orally in amounts between 5 and 25 mg per day.

Today Mesterolone is not prescribed for woman and its prescription is even exclusive for male patients. Mesterolone is sold for example under the commercial name Proviron. Mesterolone administration is prohibited for woman by manufacturers because of risks of virilisation as opposed to what the invention shows when prescribed in physiological amount. Androgens deficiencies (testosterone and dihydrotestosterone) in woman lead to ageing diseases.

### ANDROGENS IN WOMAN.

The technical term "androgen" includes true androgens such as testosterone and dihydrotestosterone (DHT) active on their receptors, but also precursors of androgens such as the dehydroepiandrosterone (DHEA), dehydroepiandrosterone sulphate (SDHEA), and A4-androstenedione, as androgens metabolites among which androstanediol, androsterone and their glucuronides derivatives (which are the principal metabolites' representatives).

### ORIGINS OF ANDROGENS PRODUCTION IN WOMAN

Androgens' are produced daily: In the ovaries, In the adrenals, In peripheral tissues and constitute the total androgens' production. With ageing the daily androgen's production varies in those three pathways. When the ovaries' production of androgens decreases for one or another reason (ageing, oophorectomy, menopause) the total pool of androgens is diminished. When the total daily production is sufficient they are no symptoms of androgens' deficiencies. This explains why women don't suffer from androgens' deficiencies at the same age.

### DAILY PRODUCTION OF ANDROGENS During the normal menstrual cycle

**Table 1**

| Testosterone and Estradiol productions in µg/day during the normal menstrual cycle | | |
|---|---|---|
| | Estradiol | Testosterone |
| Proliferative phase | 40 | 200 |
| Secretory phase | 200 | 200 |

According to E-E Baulieu and Paul A. Kelly. Hormones p.425, Hermann publishers, 1990 (1) Before menopause normal woman produces each day 5 times more testosterone than estradiol in the proliferative phase and the same quantity in the secretory phase (1).

**Table 2**

| Androgens' precursors productions in µg/day during the normal menstrual cycle | | | |
|---|---|---|---|
| | DHEA | DHEAS | Androstenedione |
| Proliferative phase | 5000 | 15000 | 4000 |
| Secretory phase | 5000 | 15000 | 4000 |

According to E-E Baulieu and Paul A. Kelly. Hormones p.425, Hermann publishers, 1990 (1)

### TESTOSTERONE, DIHYDROTESTOSTERONE AND MESTEROLONE IN WOMAN

In woman plasma dihydrotestosterone represents almost 50 % of testosterone levels

**Table 3**

| Woman before menopause: Plasmatic hormonal concentrations in ng/ml | |
|---|---|
| Testosterone | 0,57 ± 0,19 ng/ml |
| Dihydrotestosterone (DHT) | 0,27 ± 0,06 ng/ml |

According to E-E Baulieu and Paul A. Kelly. Hormones p.432. Hermann publishers, 1990 (1). Depending from its molecular structure Mesterolone (see appendix chemical formula 1) has properties similar to those of testosterone (see appendix chemical formula 2). Depending from its molecular structure Mesterolone has properties similar to those of dihydrotestosterone (see appendix chemical formula 3).

### ANDROGENS' DEFICIENCIES BEFORE MENOPAUSE

- **Testosterone secretion decreases regularly from the age of 20 in woman:** The expected Testosterone concentration in the blood of a woman of 40 would be about half that of a woman of 21(2).

### • ORAL CONTRACEPTIVE TROUBLES

Women using OCs (OC=Oral contraceptive) had significantly lower serum androgen levels compared to naturally cycling women and free testosterone levels displayed an inverse relation to breast epithelial proliferation (3.1).

OC use, however, has been associated with women's sexual health complaints and androgen insufficiency. OC use is associated with a decrease of androgen ovarian synthesis and an increase in the production of sex hormone-binding globulin (SHBG) even months after the stop of OC. Troubles may persist after stopping the OC (4).

**Table 4**

| Consequences of oral contraceptive troubles with decrease of androgen ovarian synthesis | |
|---|---|
| Thrombosis of the Deep Veins (3) | Hypertension (14/9) after 5 years of continuous use (5% of the cases) (3) |
| Pulmonary embolism (risks x 2 to 12).(3) | Disturbances of the plasmatic lipoproteins. (3) |
| Cerebral thromboembolism (risks x 3 to 9) (3) | Resistance to insulin. (3) |
| Cerebral haemorrhage (risks x 2) (3) | |
| Breast cancer risk | (3.1)(3.2)(3.3) |
| Sexual health complaints | (4) |

### • BILATERAL OOPHORECTOMY

### • PREMENOPAUSE

In this condition balance between estradiol and progesterone could be impaired leading to fibromyoma of the uterus, spotting and bleeding. Androgen's deficiency will be also considered in those cases.

Mesterolone therapy will benefit to all those cases with persisting troubles due to the decreased testosterone and dihydrotestosterone secretions.

### ANDROGENS' DEFICIENCIES AFTER MENOPAUSE

### •THE MENOPAUSE

The word "menopause" goes back to 1823. According to the dictionary this term means the end of the ovarian function characterized by the stop of ovulation and the menstrual haemorrhages (which are physiological changes). A second meaning means the time when the menopause occurs (or more usually "the female climacteric". These vague definitions do not make possible to identify a disease and consequently its treatment. It is impossible to understand aging diseases if the menopause phenomenon is not understood first. The menopause is a state of aging. The menopause presents obvious signs: the stopping of ovulation and the cessation of menses: those conditions are physiological and are not a disease. The random administration of estrogens associated or not with progesterone or progestogens HRT (hormonal replacement therapy) are dedicated to failure and have even noxious effects.

### FAILURE OF "HORMONAL TREATMENTS" (HRT = Hormonal Replacement Therapy) AMONG MENOPAUSED WOMEN

The "WOMENS' HEALTH INITIATIVE" is a15-year project involves over 161,000 women ages 50-79, and is one of the most definitive, far reaching programs of research on women's health ever undertaken in the U.S. (http://www.whi.org/). Results of the first HRT study on "Risks and Benefits of Estrogen Plus Progestin in Healthy Postmenopausal Women" were published in 2002 (JAMA. 2002;288:321-333) (5).

The conclusions about this randomized clinic al trial of great scale relating to 16,608 old women from 50 to 79 years treated by Progestin (2.5 mg of medroxyprogesterone acetate) and estrogens (0,625 Mg of combined estrogens "equine") were: (table 5). The study was to proceed until 2005. It was stopped after a 5.2 years average of follow-up on July 9th, 2002. It was decided to continue the treatment containing estrogens alone among women with prior hysterectomy http.//www. whi.org/findings/ht/ealone_stroke.php

**Table 5**

| WOMEN TAKING ESTROGEN PLUS PROGESTIN RELATIVE TO PLACEBO JAMA. 2002; 288:321-333 (5) |
|---|
| Stroke rates increased by 41% |
| CHD (coronary heart disease) events was increased by 29% |
| Venous thromboembolism (VTE) 2-fold greater |
| Total cardiovascular disease increased by 22% |
| Breast cancer increased by 26% |

### WOMEN WITH PRIOR HYSTERECTOMY aged 50-79 years

The Women's Health Initiative (WHI) Estrogen Alone (E-Alone) Trial was designed to assess the health benefits and risks of estrogen use in healthy postmenopausal women. In the WHI E-Alone Trial, 10, 739 women with prior hysterectomy, aged 50-79 years, were assigned to take either estrogen alone (conjugated estrogens [Premarin®]) or inactive (placebo) study pills. The National Institutes of Health stopped the E-Alone Trial ahead of schedule in February 2004 primarily because of an *increased stroke risk for women taking study pills with estrogen alone

### The pharmaceutical industry

- Even after the Women's Health Initiative (WHI) found that the risks of menopausal hormone therapy (hormone therapy) outweighed benefit for asymptomatic women, about half of gynaecologists in the United States continued to believe that hormones benefited women's health. The pharmaceutical industry has supported publication of articles in medical journals for marketing purposes (6).
- In June2011the U.S. Supreme Court refused to hear a Pfizer Inc. Unit' s appeal of a $58 million award in a case against Premarin(estrogens)and Prempro (estrogens plus medroxyprogesterone acetate)menopause drugs.

Three Nevada women who contracted breast cancer after taking the company's menopause drugs were awarded the amount in a 2007 case.
- The rebuff leaves the amount as the largest to be upheld on appeal in thousands of hormone-replacement drug suits. Over six million women took Prempro and other menopause drugs before a 2002 study pointed out their links to cancer. At one point Pfizer and his units faced more than 10,000 claims, according to lawyers for former users

### • THE MENOPAUSE DISEASE

### THE GEORGES DEBLED'S DEFINITION OF MENOPAUSE DISEASE:

To understand this disease of ageing it is advisable to establish a precise definition of it. Georges Debled MD. PhD. propounds the following definition:
The menopause disease is the whole of the physiopathological and psychopathological modifications brought out by the stop of the ovarian androgens' production.

### CAUSE

The ovaries secrete estradiol, progesterone and testosterone. The cessation of estradiol and progesterone are linked with the stop of ovulation and of menstrual hemorrhages which are physiological changes. The drastic reduction in the secretion of androgens hormones by the ovaries (at an age where the production of androgens by the suprarenal glands is already decreased) is generally not taken into account and brings about the menopause disease.

Blood rates of ovarian hormones in woman before menopause.

Estradiol: its blood rate varies from 0.06 nanograms per milliliter in the proliferative part of the menstrual cycle towards 0.2 nanograms per milliliter in the secretory part of the menstrual cycle. Testosterone: its rate is on average of 0.57 + 0,19 nanograms per milliliter throughout all ovarian cycle and dihydrotestosterone levels are commonly the half of the testosterone rates in healthy women (1- p.432). Progesterone: its blood rate varies from 0.3 to 1.5 nanograms per milliliter (in the proliferative part of the menstrual cycle towards 3 to 20 nanograms per milliliter in the secretary part of the menstrual cycle.

**Table 6**

| Woman before menopause: Plasmatic hormonal Concentration in ng/ml | | | | |
|---|---|---|---|---|
| | Estradiol | Progesterone | Testosterone | DHT |
| Proliferative phase | 0.06 | 0,3-1,5 | 0.57±0,19 | 0,27± 0.06 |
| 24 hours before ovulation | 0.6 | | | |
| Secretory phase | 0.2 | 3-20 | 0.57±0,19 | 0,27±0.06 |

According toE-E Baulieu and Paul A. Kelly. Hormones p.425, 432. Hermann publishers, 1990 (1)

### IN WOMAN CONCENTRATION OF TESTOSTERONE IN PLASMA IS ALWAYS GREATER THAN ESTRADIOL CONCENTRATION (1)

The plasma concentration of testosterone is + or - 10 fold the level of estradiol during the proliferative phase and + or - 3 fold the level of estradiol during the secretory phase. Serum levels of testosterone increases during ovulation (Direct RIA) in normal menstrual cycle. Massafra C. and all. 1999 (7)

### Daily production of ovarian hormones

Estradiol: its daily production varies from 40 micrograms per day in the proliferative part of the menstrual cycle around 200 micrograms per day in the secretory part of the menstrual cycle. Testosterone: its daily production is of 200 micrograms throughout all ovarian cycle. The progesterone: its daily production is of 4,200 micrograms during the proliferative phase of the ovarian cycle and of 42,000 micrograms in the secretory part of the menstrual cycle.

**Table 7**

| Woman before menopause: Normal daily production (µg/j) | | | |
|---|---|---|---|
| | Estradiol | Progesterone | Testosterone |
| Proliferative phase | 40 | 4200 | 200 |
| 24 hours before ovulation | | | |
| Secretory phase | 200 | 42000 | 200 |

According toE-E Baulieu and Paul A. Kelly Hormones, p.425. Hermann publishers, 1990 (1)

The secretions of these three hormones stop in the ovaries at the time of the menopause. The secretory and proliferative cycle controlled by estradiol and progesterone intended to fertilize ovules does not exist any more after the "suspension of the menses". One can logically wonder which reason would justify a systematic replacement of these hormones except the fact of wanting to prolong in time an ovarian cycle become useless in the absence of ovulation? The total production of testosterone during a menstrual cycle is more important in quantity compared with the production of estradiol. Consequently one is in right to ask for why estradiol substitution was proposed in the past by neglecting the production of testosterone? The sharp fall of front testosterone secretion at the time and after the stop of menses is responsible for most of the disorders caused by the menopause disease.

### CONSEQUENCES AND SYMPTOMS

The reduction in androgens' production causes in woman to different degree:
functional symptoms : hot flashes, irritability, intestinal distension, swollen legs.
local consequences:
sclerosis of bladder neck (chronic cystitis, incontinence, urgencies) sclerosis of vulva (painful or
difficult copulation).
general consequences :
lipids' disorders
vascular disorders
weakness
hyper coagulation
venous thromboses
rheumatic problems
nervous breakdown
cerebral involution
Alzheimer's disease

These consequences are wrongfully allotted to the lack of estradiol and progesterone (a polluted concept) whereas in fact they are the consequences of a lack of male hormones (testosterone for general consequences and dihydrotestosterone for local genital involution).

Previous attempts (see for example WO-A1-03/039553) to treat certain of these consequences implies complex pharmaceutical dosage forms for intravaginal administration containing at least two therapeutic agents selected from
(a) steroidal and nonsteroidal estrogens (about 20 estrogens are cited),
(b) an androgen (mesterolone is one of the 15 androgens cited), and
(c) an antimuscarinic.

In CAVADAS LF et al. "management of menopause in primary health care", Acta Medica Portuguesa 2010, Centro Editor Livreiro da Ordem dos Medicos PRT, vol. 23, No. 2, March 2010, pages 227-236 (XP00916886 - ISSN: 0870-399X), various treatments of symptoms associated with menopause are described. In particular, it is to be noted that this paper indicates that the lack of estrogen is the cause for all these symptoms (and not the lack of dihydrotestosterone) and that these symptoms must be treated by administering the missing estrogens.

TRAISH AM et al., "Testosterone therapy in women with gynecological and sexual disorders: a triumph of clinical endocrinology from 1938 to 2008", Journal of sexual medicine, Blackwell Publishing Ltd., Oxford Publishing Ltd., Vol. 6, No.2, 1 January 2009, pages 334-351, ISSN 1743-6095, describes the use of testosterone or methyltestosterone in patients with gynecological disorders, in particular, for treating symptoms associated with menopause.

General Symptoms are the same in man suffering from andropause disease described for the first time by Georges Debled in 1988. See: http://www.hmseurope.com/andropause book présentation.htm and http://www.hmseurope.com/andropause disease.htm .

The masculine genitalia of woman are:
1. The clitoris and its foreskin
2. The labia majora
3. The bladder neck (= Inner part of urethra = Homology with prostate musculature in Man)

Local Symptoms resulting from of masculine genitalia involutions are:
chronic cystitis, incontinence, urgencies. (sclerosis of bladder neck)
painful or difficult copulation (sclerosis of vulva) and are consequences of a lack of dihydrotestosterone production (lack of testosterone leads to a decreased production of dihydrotestosterone).

Balanced treatment with male hormones (mesterolone) is indicated in menopause disease as in andropause disease. This fact is ignored so that the administration of estradiol (or estrogens) associated or not with progesterone or progestogens doesn't constitute the treatment for the menopause disease whose I gave the definition (androgens' deficiencies). One can even wonder whether the "traditional" treatments of hormonal replacement therapy (HRT) do not worsen the state of good health. See http://www.whi.org

### TREATMENT OF IN ANDROGENS' DEFICIENCIES AND OF MENOPAUSE DISEASE (Dr Georges Debled's definition) WITH MESTEROLONE

Orally administration of mesterolone in amounts between 5 milligrams and 25 milligrams per day constitutes the specific treatment. If there is no symptom, there is no disease. If there is no disease no curative treatment is necessary. Today Mesterolone is not prescribed for woman and its prescription is even exclusive for male patients. The North American Menopause Society (NAMS) defines Androgens as: "a group of hormones that promote the development and maintenance of male secondary sex characteristics and structures. They are produced in smaller quantities in women and are important in the synthesis of estrogen. They also play a role in sexual function, muscle mass and strength, bone density, distribution of fat tissue, energy, and psychological well-being. With women, the major androgens are produced in the ovaries and adrenal glands and include testosterone, androstenedione, and dehydroepiandrosterone (DHEA). Also available as prescription and non-prescription therapies, but not government approved for use in women".

Mesterolone administration is prohibited for woman by the manufacturers because of risks of virilisation as opposed to what the invention shows with physiological doses. The patent covers all indications for androgens with women

It is here question to industrialize mesterolone at very another end that for which it was intended by manufacturing tablets of 5 and 10 milligrams sectile in two parts what makes it possible to cover a large range of therapeutic amounts.

Mesterolone is a hormone prescribed for man to compensate a lack of production of androgens. Used for man since 1967 it is henceforth in the public domain. Its manufacturing technique is known. No harmfulness was described to date.

Before menopause woman secretes each day of the cycle 0.2 milligrams of testosterone = 200 micrograms or 200,000 nanograms or 200,000,000 picograms. Mesterolone makes it possible to replace androgens whose production is strongly decreased in woman with menopause disease.

The invention relates to all the therapeutic ones containing mesterolone for woman; that the mesterolone is used only or in partnership with all the pharmaceutical compositions using estrogens alone or in partnership with progesterone or progestogens ones.

Mesterolone prescription is the right way to treat premenopause problems in addition to compositions using estrogens alone or in partnership with progesterone or progestogens ones. Mesterolone balances the properties of estrogens (prescribed with or without progestogens) impairing and excess of actions o their hormonal targets.

Magistral prescription is not prohibited. However the interest of the industrialization for women is evident. Making also dermal patches and pills with long lasting effects.

### WHY IS MESTEROLONE THE TREATMENT FOR ANDROGEN'S DEFICIENCIES IN WOMAN?

Mesterolone cannot be aromatized in estradiol (contrary to testosterone). Its methyl radical inserted on Carbon 1 of the testosterone confers this property. At physiological doses Mesterolone does not influence the secretion of the pituitary gland so that the secretion of LH is not modified (contrary to testosterone). Mesterolone prescribed in small amounts adds its effects to those of testosterone secreted by the organism. With the prescribed physiological pharmacological amounts doping is impossible and the overdose too. Mesterolone is prescribed orally in amounts varying between 5 milligrams and ten milligrams per day approximately. A substitution amount of 25 milligrams per day can be considered. The secretion of LH by the pituitary gland is not inhibited (contrary to testosterone).

Mesterolone molecular structure has characteristics of dihydrotestosterone (DHT) which is directly effective on the masculine sex organs of women (clitoris, labia majora and bladder neck) and on brain tissue (preventing Alzheimer's disease) (15). Mesterolone can be prescribed alone.

To restore balance with the androgens Mesterolone can be associated with certain compositions containing estradiol and of progesterone (or progestogens) in cases or these treatments would justify themselves. Any woman secretes each day 0.2 milligrams of male hormones. This balance is essential to the good performance of the hormonal targets. It is most probably this lack of balance by default of androgens which explains the disasters revealed by the "WOMENS' HEALTH INITIATIVE" in addition to inadequate diagnosis and treatments whith estradiol and progestogens. http://www.whi.org

### NON-EXISTENT RISKS OF VIRILISM WITH MESTEROLONE TREATMENT

The treatment of androgen's deficiency simply consists in replacing missing secretions of testosterone (and dihydrotestosterone) thanks to mesterolone properties. In this case the woman finds simply her former physiological state and prevents the disastrous consequences described above. Mesterolone used in small amounts allows that. Virilism secondary to an excessive administration of mesterolone is the consequence of a doping which must be avoided in all cases. Virilism doesn't exist at physiological doses

### ANDROGEN'S scientific BIOLOGIGAL DEFICIT DIAGNOSIS BACKGROUND

About determination of testosterone and dihydrotestosterone in serum:
The GC-MS (Gaz Chromatography- Mass Spectrometry) is the most precise method (Taieb and all, 2003) (8).

Direct RIA studies are significant (ACS 180 from Bayer Diagnostics) (Davison SL and all. 2005) (9) Androgen glucuronides, instead of testosterone, are interesting markers of androgenic activity in women(Labrie F. and all.2006) (10).

In the Georges Debled's study the scientific biological diagnosis of androgen's deficiencies is made on the total "pool" of androgens(RIA) in men since 37 years and in women since 10 years concluding that the level of androgens' daily production is a key diagnosis (see below). This method led Georges Debled MD.PhD. to the concept of andropause disease (which is different from hypogonadism) which treatment is made with mesterolone (See: www.hmseurope.com/andropause disease.htm)

The 10 years' study on androgens' deficiencies in women is founded on RIA analyses made by a reference laboratory (Liege University, CHU, Liege, Belgium). The Georges Debled's androgens' pool study reflects the daily production of androgens:

**Table 8**

| Androgens in serum (RIA) | |
|---|---|
| Total testosterone | DHEA |
| Dihydrotestosterone | DHEA Sulfate |
| Androstanediol glucuronide (Androsterone glycuronide) | Δ4-Androstènedione |
| Metabolites in urine over 24 hours | |
| Total 17 ketosteroids | |
| Complete Chromatography of 17 ketosteroids | |
| Androstanediol glucuronide | |
| FSH, LH and estradiol in serum | |

### ANDROGEN'S DEFICIT AND CLINICAL DIAGNOSIS

The reduction in androgens' production causes in woman to different degree:
functional symptoms: hot flashes, irritability, intestinal distension, swollen legs
local consequences:
sclerosis of bladder neck (chronic cystitis, incontinence, urgencies) sclerosis of vulva (painful or
difficult copulation)
general consequences :

**Table 9**

| | |
|---|---|
| lipids' disorders | rheumatic problems |
| vascular disorders | nervous breakdown |
| Weakness | cerebral involution |
| hyper coagulation | Alzheimer's disease |
| venous thromboses | |

### CLINICAL AND BIOLOGIGAL DIAGNOSIS OF ANDROGEN'S DEFICIT

The Georges Debled's study concludes that symptoms of androgens' deficiencies in women are correlated with a low daily production of androgens reflected by low serum androgens' levels and low levels of metabolites in serum and urine over 24 hours (the androgens' pool study) leading to treat those women successfully with mesterolone since 10 years with spectacular results and without any side effect.

### THE 2012 GENERAL PRACTICE FOR ANDROGENS' DEFICIT IN WOMAN

If there is no symptom there is no disease. And if there is no disease no treatment is necessary. The global androgens' pool was evaluated for research purpose. A simplified biological diagnosis is often enough in general practice

More than 50 women are followed since 10 years. Our 10 years experience with mesterolone prescription for androgen's deficit in general practice will continue as an increased number of women ask to follow this treatment. Hormonal substitution is founded on the clinical story and on a simplified hormonal and biological check-up (which is not expensive) for each case individually (11).

Each general practitioner, each gynaecologist, each doctor will be aware of:
The clinical diagnosis of androgens' deficiencies in women.

The simplified and non-expensive biological diagnosis of androgens' deficiencies in women.

The successful and cheap treatment of androgens' deficiencies in women with mesterolone.

**Table 10**

| Testosterone and dihydrotestosterone (DHT) deficiencies produce | | |
|---|---|---|
| | Symptoms | Pathology |
| Functional symptoms | hot flashes, intestinal distension, swollen legs | Weakness of all smooth musculatures of arteries, veins and bowel (6,5 meters of smooth musculature for the small intestine; 1,5 meters of smooth musculature for the large intestine) |

**Table 11**

| Testosterone and dihydrotestosterone (DHT) deficiencies produce | | |
|---|---|---|
| | Symptoms | Pathology |
| Local consequences | chronic cystitis, incontinence, urgencies incontinence) | sclerosis and inflammation of bladder neck |
| | dyspareunia (painful or difficult copulation) | sclerosis of vulva |
| Cardio vascular risk | lipids' disorders vascular disorders hyper coagulation venous thromboses diabetes 2 | Low testosterone levels predict all-cause mortality and cardiovascular events in women: a prospective cohort study in German primary care patients (12) |
| Locomotor system disabilities | weakness muscular atrophy rheumatic problems | Women with higher free T levels have greater lean body mass consistent with the anabolic effect of T. Bone density is associated with T levels (13). |
| | | Connective Tissue Changes of Aging in postmenopausal woman (14) |
| Cerebral consequences | Irritability nervous breakdown cerebral involution Alzheimer's disease | Comparing women with and without AD (Alzheimer disease), Female brains levels of estrogens and androgens are lower in AD cases aged 80 years and older. |
| | | Age-related depletion of androgens and estrogens in women may be relevant to development of AD (15) |

### THE MASCULINE GENITALIA OF WOMAN ARE:

The clitoris and its foreskin (homology penis and foreskin)
The labia majora (homology scrotum)
The bladder neck (homology prostate)

The masculine genitalia of woman depend of the strong male hormone (dihydrotestosterone)like male genitalia.

### SCLEROSIS OF VULVA (LABIA MAJORA), AND INVOLUTION OF THE CLITORIS

Difficult copulation, poor sensitivity of the clitoris and consequently lack of libido are the consequences of a lack of the strong male hormone (dihydrotestosterone) production which induces an involution of the masculine genitalia of woman. Those sexual problems are improved with mesterolone which molecular structure is similar to the dihydrotestosterone structure.

### SCLEROSIS AND INFLAMMATION OF BLADDER NECK

Incontinence, urgencies, chronic cystitis and devastating disorder of interstitial cystitis can be the consequences of a bladder neck sclerosis and inflammation (see drawings) .

Numerous bladder neck scleroses in women are the consequences of androgens' deficiencies (mainly dihydrotestosterone) before menopause (oral contraceptive) or after menopause (menopause disease: see above).

Those bladder neck consequences (Incontinence, urgencies, chronic cystitis and devastating disorder of interstitial cystitis) due to androgens' deficiencies may improve with mesterolone. Results can be spectacular.

As urologist Georges Debled MD. PhD. has an experience about bladder neck sclerosis (and its endoscopic surgery) in woman since 40 years. The last 10 years he discovered the role of dihydrotestosterone on the woman' s bladder neck and the benefit effect of mesterolone on this pathology.

There exists no description of mesterolone effect on bladder neck in the world literature. Those findings are disclosed here for the first time for this patent.

### BLADDER NECK SCLEROSIS AS CONSEQUENCE OF ANDROGEN'S DEFICIENCIES IN WOMEN

As requested for the patent attached files of drawings are in black and white. For better understanding, if necessary, file with same drawings in colors can be asked to georgesdebled@adslmail.es

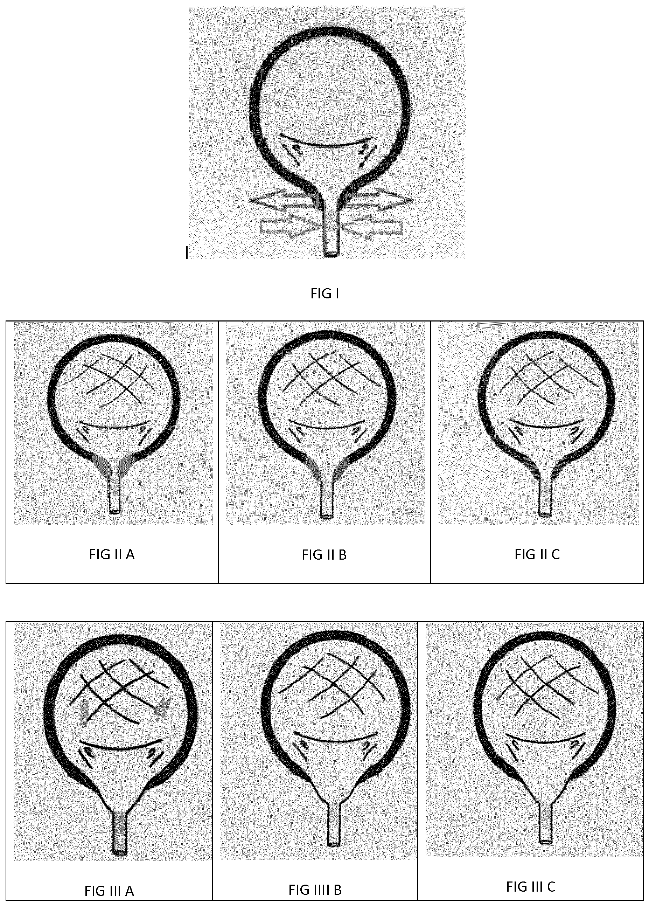

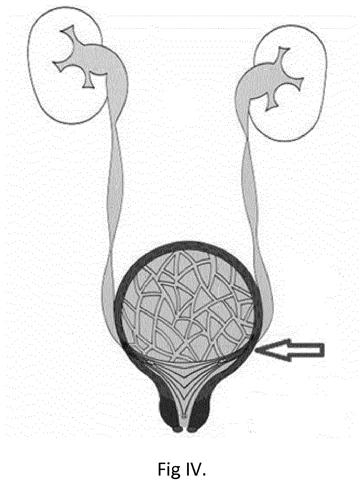

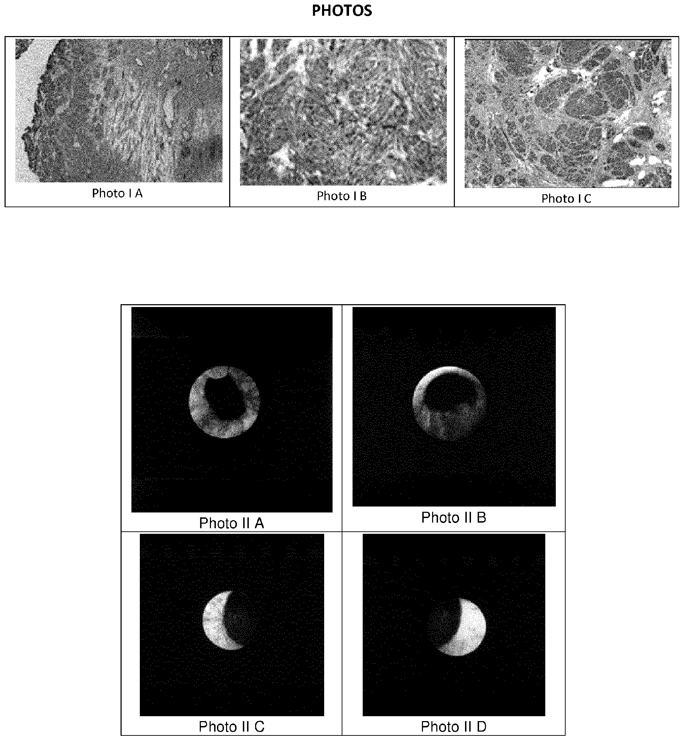

URETHRA AND BLADDER NECK IN WOMAN Urethra is constituted from 3 parts approximately from same length (1 centimeter in adult woman) (FIG 1):
The inner part (bladder neck) is constituted by a fibromuscular tissue in continuity with the muscular tissue of the bladder.

The medium part is constituted by the sphincter
The external part is a conduct.

### THE ROLE OF THE NORMAL BLADDER NECK

The normal bladder neck musculature opens actively the posterior urethra when voiding (FIG 1. divergent arrows).

The bladder neck is known in the medical literature, even in medical books of great value, as the inner sphincter. As a sphincter is a muscular structure contracted around a natural orifice (FIG 1. convergent arrows) the bladder neck is not a sphincter. Surrounding a natural orifice, it is not contracted and its contraction leads to the opening of its natural orifice on the contrary of a sphincter!

### FIBROSIS OF THE BLADDER NECK IN WOMAN

Fibrosis of the bladder neck can be congenital but a great number of cases are acquired during life. When opening the bladder neck doesn't open well and its maximum diameter is often less than 1 centimeter(FIG II A, FII B, FIG II C).
FIG II A: Bladder neck sclerosis (in grey) with narrowing of the urethra.
FIG II B: Bladder neck sclerosis (in grey) without narrowing of the urethra.
FIG II C: Moderate bladder neck sclerosis (striated in grey) without narrowing of the urethra.

### IN WOMAN: BLADDER NECK CONSEQUENCES OF ANDROGENS' DEFICIENCIES

The bladder neck is a target for male hormones (dihydrotestosterone ; homology with prostate in man). Dihydrotestosterone is locally missing during androgens' deficiencies in women: the bladder neck musculature becomes fibrous and stiff as the bladder neck musculature atrophies progressively. At the same time venous dilatations make the bladder neck congestive with and abnormal sensitivity (photo 2A and photo 2B). When there is no acute infection of urine, woman's complains are often considered as "psychotic" by doctors.

Incontinence, urgencies, chronic cystitis and devastating disorder of interstitial cystitis can be the consequences of a bladder neck sclerosis and inflammation secondary to androgens' deficiencies in women.

### MECHANIC CONSEQUENCES OF A BLADDER NECK SCLEROSIS ARE

- Hypertrophy of bladder musculature (16) or weak bladder musculature (16)
- Bad opening of bladder neck leads to secondary narrowing of terminal ureter: see arrow FIG IV.
- Hypertension in upper urinary tract (16)
- Bad function of kidneys (which are under abnormal pressure) (16)
- In summary: destruction of the urinary tract. http://www.hmseurope.com/urinary_tract_hypertension%20tests.htm

### ANATOMOPATHOLOGY OF BLADDER NECK SCLEROSIS IN WOMAN

- Histologic slide of bladder neck in woman: important sclerosis. Photo 1A.
- Histologic slide of bladder neck in woman: normal musculature. Photo 1 B
- Histologic slide of bladder neck in woman: sclerosis (light grey). Photo 1 C.

### INCIDENCE OF ANDROGENS' DEFICIENCIES ON WOMAN'S BLADDER NECK

Venous dilatations make the bladder neck congestive with and abnormal sensitivity.

When there is no acute infection of urine, woman's complains are often considered as "psychotic" by doctors. Mesterolone only treatment has cured spectacularly a woman with proved androgens' deficiencies (The Georges Debled's study) who's bladder neck was congestive with abnormal sensitivity. Endoscopic surgery was postponed thanks the mesterolone treatment. This woman is free of symptoms since 4 years.
- In woman: endoscopic view of bladder neck sclerosis with congestion. Photo II A.
- In woman: endoscopic view of bladder neck sclerosis with inflammatory polyps. Photo IIB.
- In woman: Right view of bladder neck after endoscopic resection. Photo IIC.
- In woman: Right view of bladder neck after endoscopic resection. Photo IID.

### EVOLUTION OF LOWER URINARY TRACT INFECTION AFTER REMOVE OF BLADDER NECK SCLEROSIS IN WOMAN

- Immediately after bladder neck resection of fibrous tissue, inflammation tissue may remain in the bladder wall or in the anterior urethra (in grey). FIG III A.
- Immediately after bladder neck resection of fibrous tissue, inflammation tissue may remain in the anterior urethra (in grey). FIG III B
- After bladder neck resection of fibrous tissue inflammation disappears generally completely within 3 months. FIG III C.

The Georges DEBLED's study ON MENOPAUSE DISEASE is still confidential
- It was not possible to publish those conclusions before because consequences of menopause were wrongfully allotted to the lack of oestradiol and progesterone.

This polluted concept is still considered as a "dogma" by doctors in general.

Even more import and prescription of mesterolone are not permitted by the US government. Manufacturers reserved mesterolone only for men.

The study was conducted in confidence by Georges Debled MD. PhD. during the last ten years after the first conclusions of the WHI study in 2002. JAMA. 2002 Jul 17;288(3):321-33(5)

This confidential study was possible in Europe where mesterolone is available.

After the conclusions of the WHI study to date and a recent judgment (June 2011) of the U.S. Supreme Court Georges Debled MD. PhD. discloses here for the first time the conclusions of his study on mesterolone treatment for androgens' deficiencies in woman which is the real solution in the world for women with androgens' deficiencies before and after menopause. This study has not been published in a scientific revue nor in a magazine. It has not been presented in any medical congress or conference.

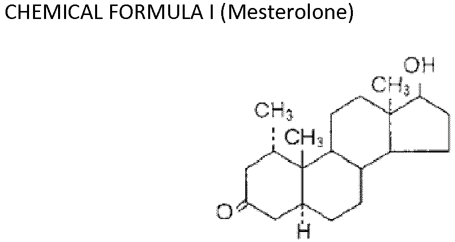

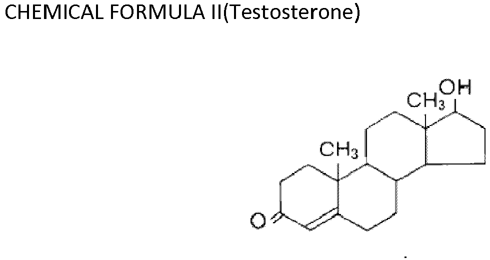

### CHEMICAL FORMULA III (Dihydrotestosterone) (DHT)

### References

1. Hormones. E-E Baulieu and Paul A. Kelly. Hermann publishers 1990)
2. Twenty-four hours mean plasma testosterone concentration decline in non premenopausal women. Zumoff B, Strain GW,Miller LK, Rosner W. J Clin Endocrinol Metab, 1995, 80:1429-1430
3. Consequences of oral contraceptive troubles with decrease of androgens ovarian synthesis. Harrison's Principes de medicine interne - 331 : Maladies de l'ovaire et de l'appareil genital 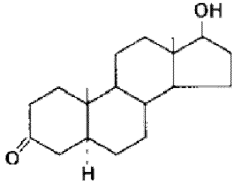 féminin: p. 1832- 1833, 1988.
   3.1.Effects of oral contraceptives on breast epithelial proliferation. Isaksson E, von Schoultz E, Odlind V, Soderqvist G, Csemiczky G, Carlstrom K, Skoog L, von Schoultz B. Department of Oncology, Radiumhemmet, Karolinska Hospital, Stockholm, Sweden Breast Cancer Res Treat2001 Jan; 65(2):163-169
   3.2 Oral contraceptive use as a risk factor for premenopausal breast cancer: a meta-analysis. Kahlenborn C, Modugno F, Potter DM, Severs WB. Mayo Clin Proc. 2006 Oct;81 (10):1290-302 .
   3.3. Effect of Depo-Medroxyprogesterone Acetate on Breast Cancer Risk among Women 20 to 44 Years of Age.Christopher I. Li, Elisabeth F. Beaber, Mei Tzu Chen Tang,Peggy L. Porter, Janet R.Daling, Kathleen E. Malone. Cancer Res; 72(8); 2028-35.2012
4. Impact of Oral Contraceptives on Sex Hormone-Binding Globulin and Androgen Levels: A Retrospective Study in Women with Sexual Dysfunction.Panzer et al. The Journal of Sexual Medicine, January 2006;3:p. 104-113
5. Risks and benefits of estrogen plus progestin in healthy postmenopausal women: principal results From the Women's Health Initiative randomized controlled trial. Rossouw JE, Anderson GL, Prentice RL, LaCroix AZ, Kooperberg C, Stefanick ML, Jackson RD, Beresford SA, Howard BV, Johnson KC, Kotchen JM, Ockene J; Writing Group for the Women's Health Initiative Investigators. JAMA. 2002 Jul 17;288(3):321-33 .
6. Promotional tone in reviews of menopausal hormone therapy after the Women's Health Initiative: an analysis of published articles.Fugh-Berman A, McDonald CP, Bell AM, Bethards EC, Scialli AR.Department of Physiology and Biophysics, Georgetown University Medical Center, Washington, DC, USA.PLoS Med. 2011 Mar;8(3):e1000425. Epub 2011 Mar 15 .
7. Androgens and osteocalcin during the menstrual cycle.Massafra C, De Felice C, Agnusdei DP, Gioia D, Bagnoli F. Department of Obstetrics and Gynecology, University of Siena, Italy. J Clin Endocrinol Metab. 1999 Mar;84(3):971-4
8.Testosterone measured by 10 immunoassays and by isotope-dilution gas chromatography-mass spectrometry in sera from 116 men,women, and children.Taieb J, Mathian B, Millot F, Patricot MC, Mathieu E, Queyrel N, Lacroix I, Somma-Delpero C, Boudou P. Hormonology Laboratory, A. Béclère Hospital, 92141 Clamart, France. Clinical Chemistry 49, 1381-1395, 2003 .
9. Androgen levels in adult females: changes with age, menopause, and oophorectorny.Davison SL, Bell R, Donath S, Montalto JG, Davis SR. J Clin Endocrinol Metab, 2005, 90:3847-53 .
10.Androgen glucuronides, instead of testosterone, as the new markers of androgenic activity in women.Labrie F, Bélanger A, Bélanger R Bérubé R, Martel C, Cusan L, Gomez J, Candas B, Castiel I, Chaussade V, Deloche C, Leclaire J.. J Steroid Biochem Mol Biol, 2006, 99:182-188 .
11. Wide distribution of the serum dehydroepiandrosterone and sex steroid levels in postmenopausal women: role of the ovary?Labrie F, Martel C, Balser J. Endoceutics Inc, Quebec City, Quebec, Canada. Menopause.2011 Jan: 18 (1):30-43 .
12. Low testosterone levels predict all-cause mortality and cardiovascular events in women: a prospective cohort study in German primary care patients. Sievers C, Klotsche J, Pieper L, Schneider HJ, März W, Wittchen HU, Stalla GK, Mantzoros C.Department of Endocrinology, Max Planck Institute of Psychiatry, Kraepelinstrasse 2-10, Munich, Germany. Eur J Endocrinol. 2010 Oct;163(4):699-708. Epub 2010 Aug 4.
13. Higher serum free testosterone concentration in older women is associated with greater bone mineral density, lean body mass, and total fat mass: the cardiovascular health study. Rariy CM, Ratcliffe SJ, Weinstein R, Bhasin S, Blackman MR, Cauley JA, Robbins J, Zmuda JM, Harris TB, Cappola AR. Division of Endocrinology, University of Pennsylvania School of Medicine, Philadelphia, Pennsylvania 19104, USA. J Clin Endocrinol Metab. 2011 Apr;96(4):989-96. Epub 2011 Feb 2 .
14.Hormonal Influences Upon Connective Tissue Changes of Aging,SOBEL H. and MARMORSTON J. Institute for Medical Research, Cedars of Lebanon Hospital, and the Depart-ment of Biochemistry and Nutrition and the Department of Medicine, University of Southern California, Los Angeles, California, in: PINCUS G (ed.) Recent Progress in Hormone Research, vol. 14. Academic New York 1958.
15.Brain levels of sex steroid hormones in men and women during normal aging and in Alzheimer's disease.Rosario ER, Chang L, Head EH, Stanczyk FZ, Pike CJ. Davis School of Gerontology, University of Southern California, Los Angeles, CA 90089, USA. Neurobiol Aging. 2011 Apr;32(4):604-13. Epub 2009 May 9.
16. La pathologie obstructive congénitale de l'uretère terminal. G. Debled : p. 446- 452 ; Acta Urologica Belgica, 1971, 39, 371-465) See pathology in : http://www.hmseurope.com/Pathologie uretère terminal.pdf

**Table 11**

| **Testosterone and Dihydrotestosterone (DHT) deficiencies produce** | | |
|---|---|---|
| | **Symptoms** | **Pathology** |
| **Local consequences** | chronic cystitis, incontinence, urgencies incontinence) | sclerosis and inflammation of bladder neck |
| | dyspareunia (painful or difficult copulation) | sclerosis of vulva |
| **Cardio vascular risk** | lipids' disorders vascular disorders hyper coagulation venous thromboses diabetes 2 | Low testosterone levels predict all-cause mortality and cardiovascular events in women: a prospective cohort study in German primary care patients (12) |
| **Locomotor system disabilities** | weakness muscular atrophy rheumatic problems | Women with higher free T levels have greater lean body mass consistent with the anabolic effect of T. Bone density is associated with T levels (13). |
| | | Connective Tissue Changes of Aging in postmenopausal woman (14) |
| **Cerebral consequences** | Irritability nervous breakdown cerebral involution Alzheimer's disease | Comparing women with and without AD (Alzheimer *disease*),*Female brains* levels of estrogens and androgens are lower in AD cases aged 80 years and older. |
| | | Age-related depletion of androgens and estrogens in women may be relevant to development of AD (15) |

### THE MASCULINE GENITALIA OF WOMAN ARE:

- The clitoris and its foreskin (homology penis and foreskin)
- The labia majora (homology scrotum)
- The badder neck (homology prostate)

### The masculine genitalia of woman depend of the strong male hormone

(dihydrotestosterone) like male genitalia.

### SCLEROSIS OF VULVA (LABIA MAJORA), AND INVOLUTION OF THE CLITORIS

Difficult copulation, poor sensitivity of the clitoris and consequently lack of libido are the consequences of a lack of the strong male hormone (dihydrotestosterone) production which induces an involution of the masculine genitalia of woman.

Those sexual problems are improved with mesterolone which molecular structure is similar to the dihydrotestosterone structure.

### SCLEROSIS AND INFLAMMATION OF BLADDER NECK

Incontinence, urgencies, chronic cystitis and devastating disorder of interstitial cystitis can be the consequences of a bladder neck sclerosis and inflammation (see drawings)

Numerous bladder neck scleroses in women are the consequences of androgens' deficiencies (mainly dihydrotestosterone) before menopause (oral contraceptive) or after menopause (menopause disease: see above).

Those bladder neck consequences (Incontinence, urgencies, chronic cystitis and devastating disorder of interstitial cystitis) due to androgens' deficiencies may improve with mesterolone. Results can be spectacular.

As urologist Georges Debled MD. PhD. has an experience about bladder neck sclerosis (and its endoscopic surgery) in woman since 40 years. The last 10 years he discovered the role of dihydrotestosterone on the woman' s bladder neck and the benefit effect of mesterolone on this pathology.

There exists no description of mesterolone effect on bladder neck in the world literature. Those findings are disclosed here for the first time for this patent.

### BLADDER NECK SCLEROSIS AS CONSEQUENCE

### OF ANDROGEN'S DEFICIENCIES IN WOMEN

**As requested for the patent attached files of drawings are in black and white.** For better understanding, if necessary, file with same drawings in colors can be asked to georgesdebled@adslmail.es

**URETHRA AND BLADDER NECK IN WOMAN** Urethra is constituted from 3 parts approximately from same length (1 centimeter in adult woman) (FIG I):
- The inner part (bladder neck) is constituted by a fibromuscular tissue in continuity with the muscular tissue of the bladder.
- The medium part is constituted by the sphincter
- The external part is a conduct.

### THE ROLE OF THE NORMAL BLADDER NECK

The normal bladder neck musculature opens actively the posterior urethra when voiding (FIG I. divergent arrows).

The bladder neck is known in the medical literature, even in medical books of great value, as the inner sphincter. As a sphincter is a muscular structure contracted around a natural orifice (FIG I. convergent arrows) the bladder neck is not a sphincter. Surrounding a natural orifice, it is not contracted and **its contraction leads to the opening** of its natural orifice on the contrary of a sphincter!

### FIBROSIS OF THE BLADDER NECK IN WOMAN

**Fibrosis of the bladder neck** can be congenital but a great number of cases are acquired during life. **When opening the bladder neck doesn't open well and its maximum diameter is often less than 1 centimeter** (FIG II A, FII B, FIG II C).
FIG II A: Bladder neck sclerosis (in grey) with narrowing of the urethra.
FIG II B: Bladder neck sclerosis (in grey) without narrowing of the urethra.
FIG II C: Moderate bladder neck sclerosis (striated in grey) without narrowing of the urethra.

**IN WOMAN: BLADDER NECK CONSEQUENCES OF ANDROGENS' DEFICIENCIES**

The bladder neck is a target for male hormones (dihydrotestosterone ; homology with prostate in man).

Dihydrotestosterone is locally missing during androgens' deficiencies in women: the bladder neck musculature becomes fibrous and stiff as the bladder neck musculature atrophies progressively. At the same time venous dilatations make the bladder neck congestive with and abnormal sensitivity (photo 2A and photo 2B).

When there is no acute infection of urine, woman's complains are often considered as "psychotic" by doctors.

Incontinence, urgencies, chronic cystitis and devastating disorder of interstitial cystitis can be the consequences of a bladder neck sclerosis and inflammation secondary to androgens' deficiencies in women.

### MECHANIC CONSEQUENCES OF A BLADDER NECK SCLEROSIS ARE

Hypertrophy of bladder musculature (16) or weak bladder musculature (16)

Bad opening of bladder neck leads to secondary narrowing of terminal ureter: see arrow FIG IV.

Hypertension in upper urinary tract (16)

Bad function of kidneys (which are under abnormal pressure) (16)

In summary: destruction of the urinary tract.

http://www.hmseurope.com/urinary_tract_hypertension%20tests.htm

### ANATOMOPATHOLOGY OF BLADDER NECK SCLEROSIS IN WOMAN

Histologic slide of bladder neck in woman: important sclerosis. Photo 1A.

Histologic slide of bladder neck in woman: normal musculature. Photo 1B

Histologic slide of bladder neck in woman: sclerosis (light grey). Photo 1 C.

### INCIDENCE OF ANDROGENS' DEFICIENCIES ON WOMAN'S BLADDER NECK

Venous dilatations make the bladder neck congestive with and abnormal sensitivity.

When there is no acute infection of urine, woman's complains are often considered as "psychotic" by doctors.

Mesterolone only treatment has cured spectacularly a woman with proved androgens' deficiencies (The Georges Debled's study) who's bladder neck was congestive with abnormal sensitivity. Endoscopic surgery was postponed thanks the mesterolone treatment. This woman is free of symptoms since 4 years.

In woman: endoscopic view of bladder neck sclerosis with congestion. Photo II A.

In woman: endoscopic view of bladder neck sclerosis with inflammatory polyps. Photo IIB.

In woman: Right view of bladder neck after endoscopic resection. Photo IIC.

In woman: Right view of bladder neck after endoscopic resection. Photo IID.

### EVOLUTION OF LOWER URINARY TRACT INFECTION AFTER

### REMOVE OF BLADDER NECK SCLEROSIS IN WOMAN

Immediately after bladder neck resection of fibrous tissue, inflammation tissue may remain in the bladder wall or in the anterior urethra (in grey). FIG III A.

Immediately after bladder neck resection of fibrous tissue, inflammation tissue may remain in the anterior urethra (in grey). FIG III B

After bladder neck resection of fibrous tissue inflammation disappears generally completely within 3 months. FIG III C.

**The Georges DEBLED's study ON MENOPAUSE DISEASE is still confidential**
- It was not possible to publish those conclusions before because consequences of menopause were wrongfully allotted to the lack of oestradiol and progesterone.

This polluted concept is still considered as a "dogma" by doctors in general.
- Even more import and prescription of mesterolone are not permitted by the US government.
- Manufacturers reserved mesterolone only for men.
- The study was conducted in confidence by Georges Debled MD. PhD. during the last ten years after the first conclusions of the WHI study in 2002. JAMA. 2002 Jul 17,288(3):321-33*(5)*
- This confidential study was possible in Europe where mesterolone is available.
- After the conclusions of the WHI study to date and the recent judgment (June 2011) of the U.S. Supreme Court (17) Georges Debled MD. PhD. discloses here for the first time the conclusions of his study on **mesterolone treatment for androgens' deficiencies in woman** which is the real solution in the world for women with androgens' deficiencies before and after menopause. This study has not been published in a scientific revue nor in a magazine. It has not been presented in any medical congress or conference.

### • It is an absolute new invention.

### • FUTURE INVESTIGATIONS

**With the help of governments and of manufacturers** exhaustive studies will be made to specify contours of mesterolone as treatment for androgens' deficiencies in women.

These future studies on large scale will be conducted within the general framework of biology of ageing.

### APPENDIX

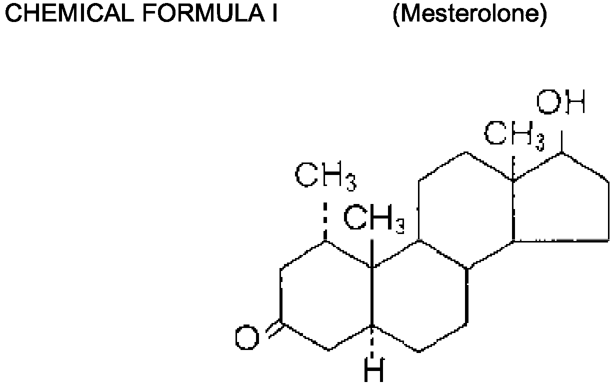

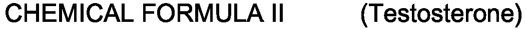

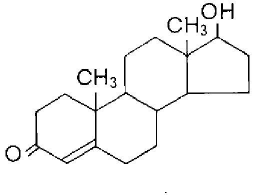

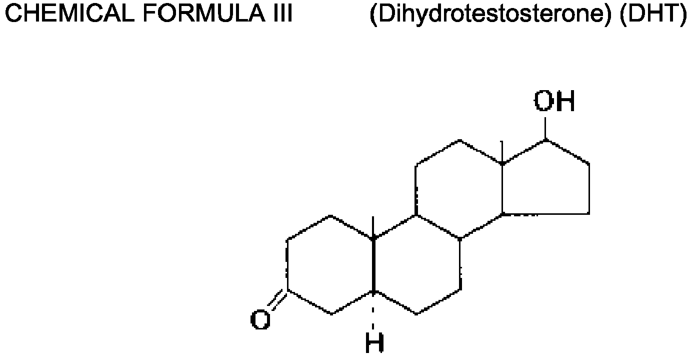

### BIBLIOGRAPHY

*1*. Hormones. E-E Baulieu and Paul A.Kelly. Hermann publishers 1990*)*
2. Twenty-four hours mean plasma testosterone concentration decline in non premenopausal women. Zumoff B, Strain GW,Miller LK, Rosner W. J Clin Endocrinol Metab, 1995, 80:1429-1430
3. Consequences of oral contraceptive troubles with decrease of androgen ovarian synthesis. Harrison's Principes de medicine interne - 331 : Maladies de l'ovaire et de l'appareil génital feminin: p. 1832-1833, 1988*.*
   3.1. Effects of oral contraceptives on breast epithelial proliferation. Isaksson E, von Schoultz E, Odlind V, Soderqvist G, Csemiczky G, Carlstrom K, Skoog L, von Schoultz B. Department of Oncology, Radiumhemmet, Karolinska Hospital, Stockholm, Sweden Breast Cancer Res Treat2001Jan; 65(2):163-169
   **3.2** Oral contraceptive use as a risk factor for premenopausal breast cancer: a meta-analysis. Kahlenborn C, Modugno F, Potter DM, Severs WB. Mayo Clin Proc. 2006 Oct;81(10):1290-302.
   **3.3.** Effect of Depo-Medroxyprogesterone Acetate on Breast Cancer Risk among Women 20 to 44 Years of Age. Christopher I. Li, Elisabeth F. Beaber, Mei Tzu Chen Tang,Peggy L. Porter, Janet R.Daling, Kathleen E. Malone. Cancer Res; 72(8); 2028-35.2012
4. Impact of Oral Contraceptives on Sex Hormone-Binding Globulin and Androgen Levels: A Retrospective Study in Women with Sexual Dysfunction.Panzer et al. The Journal of Sexual Medicine, January 2006;3:p.104-113
5. Risks and benefits of estrogen plus progestin in healthy postmenopausal women: principal results From the Women's Health Initiative randomized controlled trial. Rossouw JE, Anderson GL, Prentice RL, LaCroix AZ, Kooperberg C, Stefanick ML, Jackson RD, Beresford SA, Howard BV, Johnson KC, Kotchen JM, Ockene J; Writing Group for the Women's Health Initiative Investigators. JAMA. 2002 Jul 17;288(3):321-33.
6. Promotional tone in reviews of menopausal hormone therapy after the Women's Health Initiative: an analysis of published articles. Fugh-Berman A, McDonald CP, Bell AM, Bethards EC, Scialli AR.Department of Physiology and Biophysics, Georgetown University Medical Center, Washington, DC, USA.PLoS Med. 2011 Mar;8(3):e1000425. Epub 2011 Mar 15.
7. Androgens and osteocalcin during the menstrual cycle. Massafra C, De Felice C, Agnusdei DP, Gioia D, Bagnoli F. Department of Obstetrics and Gynecology, University of Siena, Italy. J Clin Endocrinol Metab. 1999 Mar;84(3):971-4
8.Testosterone measured by 10 immunoassays and by isotope-dilution gas chromatography-mass spectrometry in sera from 116 men,women, and children. Taieb J, Mathian B, Millot F, Patricot MC, Mathieu E, Queyrel N, Lacroix I, Somma-Delpero C, Boudou P. Hormonology Laboratory, A. Béclère Hospital, 92141 Clamart, France. Clinical Chemistry 49, 1381-1395, 2003.
9. Androgen levels in adult females: changes with age, menopause, and oophorectorny. Davison SL, Bell R, Donath S, Montalto JG, Davis SR. J Clin Endocrinol Metab, 2005, 90:3847-53.
10. Androgen glucuronides, instead of testosterone, as the new markers of androgenic activity in women.Labrie F, Bélanger A, Bélanger R Bérubé R, Martel C, Cusan L, Gomez J, Candas B, Castiel I, Chaussade V, Deloche C, Leclaire J.. J Steroid Biochem Mol Biol, 2006, 99:182-188.
11. Wide distribution of the serum dehydroepiandrosterone and sex steroid levels in postmenopausal women: role of the ovary? Labrie F, Martel C, Balser J. Endoceutics Inc, Quebec City, Quebec, Canada. Menopause.2011 Jan: 18 (1):30-43.
12. Low testosterone levels predict all-cause mortality and cardiovascular events in women: a prospective cohort study in German primary care patients. Sievers C, Klotsche J, Pieper L, Schneider HJ, März W, Wittchen HU, Stalla GK, Mantzoros C.Department of Endocrinology, Max Planck Institute of Psychiatry, Kraepelinstrasse 2-10, Munich, Germany. Eur J Endocrinol. 2010 Oct;163(4):699-708. Epub 2010 Aug 4.
13. Higher serum free testosterone concentration in older women is associated with greater bone mineral density, lean body mass, and total fat mass: the cardiovascular health study. Rariy CM, Ratcliffe SJ, Weinstein R, Bhasin S, Blackman MR, Cauley JA, Robbins J, Zmuda JM, Harris TB, Cappola AR. Division of Endocrinology, University of Pennsylvania School of Medicine, Philadelphia, Pennsylvania 19104, USA. J Clin Endocrinol Metab. 2011 Apr;96(4):989-96. Epub 2011 Feb 2.
14.Hormonal Influences Upon Connective Tissue Changes of Aging, SOBEL H. and MARMORSTON J. Institute for Medical Research, Cedars of Lebanon Hospital, and the Depart-ment of Biochemistry and Nutrition and the Department of Medicine, University of Southern California, Los Angeles, California,in: PINCUS G (ed.) Recent Progress in Hormone Research, vol. 14. Academic New York 1958.
15. Brain levels of sex steroid hormones in men and women during normal aging and in Alzheimer's disease. Rosario ER, Chang L, Head EH, Stanczyk FZ, Pike CJ. Davis School of Gerontology, University of Southern California, Los Angeles, CA 90089, USA. Neurobiol Aging. 2011 Apr;32(4):604-13. Epub 2009 May 9.
16. La pathologie obstructive congénitale de l'uretère terminal. G. Debled : p. 446- 452 ; Acta Urologica Belgica, 1971, 39, 371-465) See pathology in : http://www.hmseurope.com/Pathologie uretere terminal.pdf
17. **High Court Rejects Pfizer Hormone Therapy Lawsuit Appeal**

Date Published: Wednesday, June 22nd, 2011

The U.S. Supreme Court refused to hear a Pfizer Inc. unit's appeal of a $58 million award in a case
against Premarin and Prempro menopause drugs.

Three Nevada women who contracted breast cancer after taking the company's menopause drugs
were awarded the amount in a 2007 case.

The rebuff leaves the amount as the largest to be upheld on appeal in thousands of hormonereplacement
drug suits. Over six million women took Prempro and other menopause drugs before a
2002 study pointed out their links to cancer. At one point Pfizer and his units faced more than 10,000
claims, according to lawyers for former users.

The Nevada Supreme Court concluded jurors properly held Pfizer's Wyeth unit responsible for hiding
the breast-cancer risks of Premarin and Prempro. The original 2007 case resulted in an award totaling
$134.1 million to Arlene Rowatt, Jeraldine Scofield and Pamela Forrester. The trial judge later
reduced the verdict to $57.6 million.

Yearly sales of Wyeth's hormone-replacement drugs exceeded $2 billion before a 2002 study,
sponsored by the U.S. National Institutes of Health, suggested that women using the medicines had a
24 percent higher risk of breast cancer.

Pfizer, the world's largest drug maker, acquired Wyeth in 2009 settled a third of the pending cases
over its Prempro menopause drug. The company said last month that it set aside $772 million to
resolve claims over the medicine.

**INTERNET: Those findings will be published on: www.hmseurope.org**

## Claims

1. Mesterolone for use in the treatment and prevention treatment of disorders associated with the deficiency in dihydrotestosterone in woman, wherein the mesterolone is administered orally in amounts between 5 and 25 mg per day.

2. Mesterolone for use according to claim 1 in the treatment and prevention treatment of bladder neck fibrosis or sclerosis, chronic cystitis, incontinence and urgencies in woman.

3. Mesterolone for use according to claim 1 in the treatment and prevention treatment of dyspareunia in woman.

## Patentansprüche

1. Mesterolon für die Behandlung und vorbeugende Behandlung von Erkrankungen im Zusammenhang mit Dihydrotestosteron-Mangel bei Frauen, bei denen Mesterolon oral in Mengen zwischen 5 und 25 mg pro Tag verabreicht wird.

2. Mesterolon für Verwendung nach Anspruch 1, bei der Behandlung und präventiven Behandlung von Fibrose oder Sklerose der Blase, Harnproblemen wie Zystitis, Inkontinenz und Notfälle bei Frauen.

3. Mesterolon für Verwendung nach Anspruch 1, bei der Behandlung und präventiven Behandlung von Dyspareunie bei Frauen.

## Revendications

1. Mestérolone pour utilisation dans le traitement et le traitement préventif de désordres associés à la déficience en dihydrotestostérone chez la femme dans laquelle la mestérolone est administrée oralement en quantité comprises entre 5 et 25 mg par jour.

2. Mestérolone pour utilisation suivant la revendication 1, dans le traitement et le traitement préventif de la fibrose ou sclérose du col de la vessie, de problèmes urinaires tels que la cystite, l'incontinence et les urgences chez la femme.

3. Mestérolone pour utilisation suivant la revendication 1, dans le traitement et le traitement préventif de la dyspareunie chez la femme.
